# EUROPÄISCHE PATENTSCHRIFT

(11) **EP 1 064 064 B1**
(45) Veröffentlichungstag und Bekanntmachung des Hinweises auf die Patenterteilung: **17.11.2004**
(21) Anmeldenummer: 99913212.9
(22) Anmeldetag: 02.03.1999
(51) Int. Cl.: B01D 61/00, B01D 15/00

(54) **BESEITIGUNG VON KONTAMINATIONEN AUS BIOLOGISCHEN PRODUKTEN**
REMOVAL OF CONTAMINANTS FROM BIOLOGICAL PRODUCTS
ELIMINATION DE CONTAMINANTS CONTENUS DANS DES PRODUITS BIOLOGIQUES

(30) Priorität: 13.03.1998 DE 19810947
(43) Veröffentlichungstag der Anmeldung: 03.01.2001
(73) Patentinhaber: MERCK PATENT GmbH, 64293 Darmstadt (DE)
(72) Erfinder: MÜLLER, Egbert, D-64291 Darmstadt (DE)
(86) Internationale Anmeldenummer: PCT/EP1999/001353
(87) Internationale Veröffentlichungsnummer: WO 1999/047227

(56) Entgegenhaltungen:
- WO-A-93/08894
- WO-A-96/22316
- WO-A-97/49754
- DE-A- 19 543 371
- GB-A- 1 092 754
- US-A- 4 711 793
- US-A- 5 438 128

## Beschreibung

Die Erfindung betrifft Verfahren zur Beseitigung von Kontaminationen aus biologischen Produkten mittels Trennung unter Verwendung von Anionenaustauscher-Membranen, sowie mittels dieser Verfahren gereinigte biologische Produkte.

Hohe Anforderungen werden an die Reinheit von biologischen Produkten gestellt, insbesondere wenn sie beispielsweise für pharmazeutische Zwecke verwendet werden sollen. Dies gilt insbesonders, wenn intraperitonale oder intra-venöse Anwendungen vorgesehen sind. In diesem Zusammenhang sind insbesondere Viren, Endotoxine und Nukleinsäuren, wie DNA, als mögliche Kontaminanten von Bedeutung. Diese Kontaminanten werden als biologische Kontaminanten zusammengefaßt.

Kontaminierende Viren können beispielsweise durch chemische Inaktivierung unschädlich gemacht werden; WO 95/16 030 beschreibt dazu übliche Verfahrensvarianten. Üblich ist weiterhin die Abtrennung durch Ultrafiltration, dazu ist beispielsweise in EP 0 302 949 eine Ultrafiltrationsmembran mit spezieller Porengeometrie offenbart. Eine Abtrennung speziell von humanem Immunschwäche-Virus (HIV) an nicht porösen lonenaustauschmaterialien wird in EP 0 320 184 offenbart. Endotoxine können durch Behandlung mit einer salzhaltigen Detergenslösung entfernt werden, wie es beispielsweise in WO 95/21 179 offenbart ist. Für die Abtrennung von Endotoxinen wurden außerdem in spezieller Weise derivatisierte Membranen vorgeschlagen (WO 97/33 683). Nukleinsäuren können durch partikuläre Anionenaustauscher abgetrennt werden, wie es beispielsweise in J. Chromat. A, **658**, Seite 459 - 463 (1994) beschrieben ist.

Die US-4 711 793-A offenbart ein Verfahren zur Herstellung von Membranen, die mit Kationen beladen sind. Dabei wird die Membran, bevorzugt eine Nylon- bzw. PolyamidMembran , mit einem polymere. Agens zur Einführung kationischer Ladungen ("cationic charge modifying agent") umgesetzt. Das Agens zur Einführung kationischer Ladungen weist zumindest eine Epoxidgruppe auf und zumindest eine tertiäre oder quartäre Aminogruppe. Die Umsetzung des Agens zur Einführung kationischer Ladungen mit der Membran erfolgt, indem die Epoxidgruppen des Agens mit den Amino-, Carboxyl- und/oder Hydroxylgruppen der Membran reagieren.

Die US-5 438 128-A offenbart ein Verfahren und eine Vorrichtung zur Isolierung und Aufreinigung von Nukleinsäuren unter Verwendung einer Ionenaustausch-Membran. Es können Cellulose-, Nylon-, PVDF- oder andere Membranen eingesetzt werden, die Ionenaustauschgruppen tragen.

Aus WO 96/22 316 und WO 97/49 754 sind adsorptive Membranen auf Polyamidbasis mit verbesserten Eigenschaften bekannt. Diese verbesserten Trennmaterialien sind zugänglich durch Umsetzung der Aminogruppen eines Polyamids mit einer aminoreaktiven Verbindung oder durch Umsetzung der Carboxylgruppen eines Polyamides mit einer carboxylgruppenreaktiven Verbindung, wobei polymerisierbare Gruppen in das Basispolymer eingeführt werden. Zusätzliche Amino- beziehungsweise Carboxylgruppen können vor der Einführung der polymerisierbaren Gruppen durch Umsetzung mit Diaminen beziehungsweise Dicarbonsäuren oder Dicarbonsäurederivaten eingeführt werden. An die genannten polymerisierbaren Gruppen werden anschließend Monomere anpolymerisiert. Diese Monomeren können Separationseffektoren enthalten; es ist ebenfalls möglich, in die Monomereinheiten mittels bekannter polymeranaloger Umsetzungen Separationseffektoren einzuführen. Bezüglich der möglichen Separationseffektoren und der Herstellung von adsorptiven Membranen, die diese Separationseffektoren enthalten, wird auf die genannten Druckschriften verwiesen. Zu den in diesen Druckschriften genannten Separationseffektoren gehören auch kationische Gruppen. Die Verwendung der Anionenaustauscher-Membranen, wie sie in WO 96/22 316 und WO 97/49 754 offenbart sind, für die Entfernung von biologischen Kontaminanten aus biologischen Produkten ist Gegenstand der vorliegenden Erfindung. Für die erfindungsgemäße Verwendung sind insbesondere Anionenaustauscher-Membranen bevorzugt, die erhältlich sind durch Umsetzung eines Polyamides mit einer aminoreaktiven polymerisationsfähigen Verbindung und anschließender Polymerisation mit Monomeren, die kationische Gruppen enthalten, oder in die in polymeranaloger Reaktion kationische Gruppen eingeführt werden können.

Diese Anionenaustauscher-Membranen können beispielsweise als Flachmembranen oder als Hohlfasermembranen ausgebildet sein. Hohlfasermembranen sind in "dead end"-Konfiguration oder in "crossflow"-Konfiguration einsetzbar. Diese Ausführungsformen und deren Herstellung sind dem Fachmann bekannt.

Es wurde gefunden, daß in vorteilhafter Weise unter Verwendung der in WO 96/22 316 und WO 97/49 754 offenbarten Anionenaustauscher-Membranen die genannten Kontaminationen aus Lösungen entfernt werden können. So wurde gefunden, daß Endotoxin sowohl aus einer Pufferlösung als auch aus einer Lösung von Proteinen entfernt werden kann. Weiter wurde gefunden, daß DNA (Hind III - Fragmente von DNA des Phagen λ) von Protein abgetrennt werden kann. Weiter wurde gefunden, daß Viren aus Lösungen abgetrennt werden können, darunter auch Viren, die nach bekannten Methoden, beispielsweise durch Nanofiltration, nur unzureichend abgetrennt werden können.

Gegenstand der Erfindung sind Verfahren zur Abtrennung oder Abreicherung von biologischen Kontaminanten, wobei die aufzureinigende Probe (biologisches Produkt) mit einer Anionenaustauscher-Membran behandelt wird, wobei besagte Anionenaustauscher-Membran durch Umsetzung eines Polyamides mit einer aminoreaktiven polymerisationsfähigen Verbindung und anschließender Polymerisation mit Monomeren, die kationische Gruppen enthalten, oder in die in polymeranaloger Reaktion kationische Gruppen eingeführt werden können, erhältlich sind.

Gegenstand der Erfindung sind ferner aufgereinigte biologische Produkte erhältlich durch das erfindungsgemäße Verfahren.

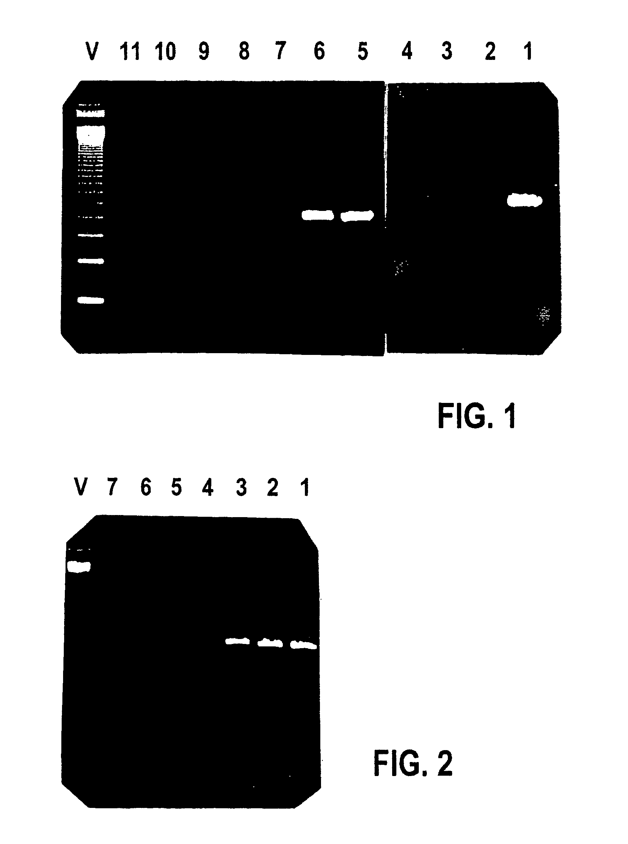

In Abbildung 1 sind PCR-Untersuchungen an verschiedenen Eluaten, die bei der Abreicherung von DNA angefallen waren, dargestellt. Abbildung 2 zeigt die Nachweisempfindlichkeit des verwendeten PCR-Protokolls auf; experimentelle Einzelheiten finden sich in Beispiel 5.

Die erfindungsgemäße Abtrennung der Kontaminanten erfolgt unter Verwendung von Puffersystemen, wie sie für die Anionenaustauschchromatographie gebräuchlich sind. Den Puffern können gegebenenfalls Neutralsalze, organische Lösungsmittel, Detergenzien und/oder chaotrope Agenzien zugesetzt werden. Derartige Zusätze sind dem Fachmann bekannt und in der Literatur beschrieben.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### Beispiele 1 - 3: Abreicherung verschiedener Viren

Die Abreicherung verschiedener Viren wurde von der Fa. Ana/lysis (Frankfurt (Main), DE) entsprechend gängiger Validierungsvorschriften ausgeführt ("Anforderungen an Validierungsstudien zum Nachweis der Virussicherheit von Arzneimitteln aus menschlichem Blut oder Plasma": Paul-Ehrlich-Institut und Bundesgesundheitsamt, Mai 1994; sowie "Note for Guidance on Virus Validation Studies": CPMP/BWP/268/95; 2. endgültige Fassung vom Februar 1996). Für die Untersuchung wurde 20 mM TRIS-Puffer (pH 7,0) mit 10 Vol.-% einer Virenstammlösung versetzt; verwendet wurden folgende Viren:
- HIV-1: Humanes Immundefizienz Virus Typ 1
- PPV: Porcine Parvovirus
- BVDV: Bovine Viral Diarrhea Virus

Weitere Angaben zu den verwandten Viren und betreffend die virussuszeptiblen Wirts- und Indikatorzellen sind in der folgenden Tabelle 1 zusammengefaßt:

**Tabelle 1:**

| | **HIV-1** | **PPV** | **BVDV** |
|---|---|---|---|
| **Stamm** | Bru | NADL-2 | RKI |
| **Herkunft** | Georg-Speyer-Haus | ATCC VR-742 | Robert-Koch-Institut |
| **Wirts-/ Indikatorzellen** | C8166-45-Zellen⁽¹⁾ | PK-13-Zellen⁽²⁾ | KL-2-Zellen⁽³⁾ |

| | | | |
|---|---|---|---|
| Anmerkungen: (1) Lymphozyten aus menschlicher Nabelschnur, mit HTLV I transformiert (Georg-Speyer-Haus; Chemotherapeutisches Forschungsinstitut, Frankfurt am Main, DE) | | | |
| (2) Zellen vom Schwein (European Collection of Animal Cells; Porton Down, Salisbury, UK) | | | |
| (3) Lungenzellen vom fötalen Kalb (Robert-Koch-Institut) | | | |

Jeweils 10 ml dieser Lösung wurden mit einer Spritze auf ein Fractoflow® 80 - 6 mm dead end Hohlfasermodul (Basismaterial Polyamid 6; Fa. Merck KGaA, Darmstadt, DE) aufgetragen. Folgende Typen von Anionenaustauscher-Hohlfasermodulen wurden verwendet:
- D: schwacher Anionenaustauscher (DEA-Typ)
- T: starker Anionenaustauscher (TMA - Typ)

Zum Vergleich dienten Module mit unmodifizierten Membranen und mit Kationenaustauscher- Membranen:
- U: underivatisiertes Basismaterial Polyamid 6
- S: starker Kationenaustauscher (Sulfo - Typ)

Virustiter wurden vor und nach der Filtration durch Endpunkttitration bestimmt. Dazu wurden serielle Dreifachverdünnungen der Probe jeweils in Achtfachansätzen hergestellt. Die verdünnten Proben wurden zu virussuszeptiblen Indikatorzellen zugefügt und die inkubierten Indikatorzellen mikroskopisch auf cytopathische Effekte (CPE) untersucht. Die statistische Auswertung erfolgte nach Standardmethoden.

### Beispiel 1: Abreicherung von HIV - 1

Entsprechend der obigen allgemeinen Verfahrensbeschreibung wurde die Abreicherung von HIV-1 untersucht; die Ergebnisse sind in Tabelle 2 zusammengefaßt:

**Tabelle 2:**

| **Modultyp** | **Abtrennung log - Stufen** |
|---|---|
| T | ≥6.08* |
| D | ≥6.08* |
| U | 3,98 |
| S. | 4,46 |

| | |
|---|---|
| * Es war kein infektiöses Material nachweisbar. | |

Bei der erfindungsgemäßen Verwendung von Anionenaustauscher-Membranen wird eine Abreicherung bis unter die Nachweisgrenze erreicht, während die Vergleichsversuche (U und S) eine um ein bis zwei Größenordnungen niedrigere Abreicherung beobachten liessen.

### Beispiel 2: Abreicherung von Porcine - Parvovirus

Entsprechend der obigen allgemeinen Verfahrensbeschreibung wurde die Abreicherung von PPV untersucht; die Ergebnisse sind in Tabelle 3 zusammengefaßt:

**Tabelle 3:**

| **Modultyp** | **Abtrennung log - Stufen** |
|---|---|
| T | ≥6.84* |
| D | ≥6.84* |
| U | -^{#} |
| S | -^{#} |

| | |
|---|---|
| * kein infektiöses Material nachweisbar; | |
| # keine Abreicherung meßbar. | |

Bei der erfindungsgemäßen Verwendung von Anionenaustauscher-Membranen wird eine Abreicherung bis unter die Nachweisgrenze erreicht, während die Vergleichsversuche (U und S) keine Abreicherung beobachten liessen. Der Porcine - Parvovirus ist sehr klein (ca. 20 nm) und sehr stabil. Dieser Virus konnte durch kein bisher bekanntes Verteilungsverfahren vollständig abgetrennt werden.

Die hier erhaltenen Ergebnisse zeigen, daß die Abtrennung der Viren sehr spezifisch nur mit den Anionenaustauschern erfolgt.

### Beispiel 3: Abreicherung von Bovine Viral Diarrhea Virus

Entsprechend der obigen allgemeinen Verfahrensbeschreibung wurde die Abreicherung von BVDV untersucht; die Ergebnisse sind in Tabelle 4 zusammengefaßt:

**Tabelle 4:**

| **Modultyp** | **Abtrennung log - Stufen** |
|---|---|
| T | 5,87 |
| D | ≥5,88* |
| U | 1,04 |
| S | 0,32 |

| | |
|---|---|
| * Es war kein infektiöses Material nachweisbar; | |

Bei der erfindungsgemäßen Verwendung von Anionenaustauscher-Membranen wird eine Abreicherung bis unter die Nachweisgrenze erreicht, während die Vergleichsversuche (U und S) eine um mehrere Größenordnungen niedrigere Abreicherung beobachten liessen.

Die hier erhaltenen Ergebnisse zeigen, daß die Abtrennung der Viren sehr spezifisch nur mit den Anionenaustauschern erfolgt.

### Beispiel 4: Entfernung von Endotoxinen

Material und Methoden :
Es wurde ein 80 - 6 mm Fractoflow® DEA - Hohlfasermodul (Fa. Merck KGaA; Darmstadt, DE) verwendet.
Die Probenaufgabe erfolgte mit einer Spritze.
Die Aktivität des Endotoxins wurde mittels des LAL-Tests bestimmt.

### a) Abtrennung aus Pufferlösung

### Probe: Endotoxin von E. coli (USP - Standard) in 20 mM Phosphatpuffer (pH7)

Die Ergebnisse der Abtrennung von Endotoxin aus Pufferlösung sind in Tabelle 5 zusammengefaßt:

**Tabelle 5:**

| **Lösung** | **Endotoxin (EU/ml)** | **Endotoxin (% der Ausgangslösung)** |
|---|---|---|
| Ausgangslösung | 1536 | 100 |
| Eluat Cyclus 1 | 3,9 | 0,25 |
| Eluat Cyclus 2 | 0,03 ^{*)} | 0,002 |

| | | |
|---|---|---|
| ^{*}) Nachweisgrenze | | |

Die Abtrennung des Endotoxins aus einer Pufferlösung gelingt durch drei Zyklen bis unter die Nachweisgrenze des LAL - Tests.

### b) Abtrennung aus proteinhaltiger Lösung

### Probe: Endotoxin von E. coli (USP - Standard; 15 724 EU/ml) und Rinderserumalbumin (BSA; 1 mg /ml) in 20 mM Phosphatpuffer (pH7)

Elution des BSA mit 0,4 M NaCl in 20 mM Phosphatpuffer (pH 7).

Die Ergebnisse der Abtrennung von Endotoxin aus einer 1 mg/ml BSA Lösung durch selektive Desorption sind in Tabelle 6 zusammengefaßt.

**Tabelle 6:**

| **Lösung** | **Endotoxin *(EU/ml)** | **Endotoxin (% der Ausgangslösung)** | **Rinderserumalbumin (BSA) (Wiederfindung %)** |
|---|---|---|---|
| Ausgangslösung | 15724 | 100 | 100 |
| Eluat Cyclus 1 | 422 | 2,7 | 95 |
| Eluat Cyclus 2 | 3,5 | 0,02 | 94 |

BSA und Endotoxin können durch selektive Desorption des Albumins bei 0,4 M Kochsalz getrennt werden.

### Beispiel 5: Entfernung von DNA

Es wurde ein 80 - 6 mm Fractoflow® DEA - Hohlfasermodul verwendet. Die Probenlösung wurde mit einer Spritze aufgegeben.
10 ml einer BSA - Lösung mit einer Konzentration von 1 mg/ml in 20 mM Tris pH 8,5 wurden mit 1 µg λ -DNA (geschnitten mit Hind III) versetzt. Nach der Probenaufgabe wurde mit 10 ml Pufferlösung (20 mM Tris pH 8,5 nachgewaschen. Das Protein wurde mit 15 ml einer 20 mM Tris - Lösung + 1 M NaCl pH 8,5 eluiert. Anschließend wurde wieder mit 10 ml eines 20 mM Tris Puffers pH 8,5 nachgewaschen.
BSA wurde unter diesen Bedingungen gebunden und konnte durch 1 M Kochsalzzusatz mit einer Wiederfindung von 97 % eluiert werden. Die DNA konnte mit 1 M Kochsalz nicht abgelöst werden und konnte erst mit 2 x 10 ml einer 1 M Natronlauge entfernt werden.
- Bahn 1 :: λ - DNA
- Bahn 2 :: Probenaufgabe
- Bahn 3 :: 20 mM Tris-Puffer (pH 8,5)
- Bahn 4 :: 1 M NaCl in 20 mM Tris-Puffer (pH 8,5)
- Bahn 5 :: 1 M NaOH
- Bahn 6 :: 1 M NaOH

Für die Bahnen 7, 8, 9, 10 und 11 wurden weitere Eluate mit 6 M Harnstoff, 20 mM Tris, 6 M Guanidiniumchlorid. 20 mM Tris, Ethanol verwendet.

In den verschiedenenen Eluaten wurde die DNA mittels PCR folgendermaßen nachgewiesen:

### Reagenzienmischung (Mix):

| | |
|---|---|
| 10 × Polymerase - Puffer | 20 µl |
| dNTPs (20 mM) | 2 µl |
| Primer λ1 (5 pmol/µl) | 8 µl |
| Primer λ2 (5 pmol/µl) | 8 µl |
| Taq-Polymerase (5U/µl) | 2 µl |
| Wasser | 80 µl |

Gemischt wurden 15 µl der obigen Reagenzienmischung und 10 µl Probe (jeweiliges Eluat; 1 - 11); anschließend wurde die Polymerase-Ketten-Reaktion (PCR) ausgeführt und die Reaktionsprodukte nach Elektrophorese nachgewiesen:

### PCR Programm:

3 min, 94 °C
10X
   15 sec, 92 °C
   15 sec, 65°C(-1°C/Cyclus)
10X
   15 sec, 92°C
   15 sec, 55°C
5 min, 72°C bis Ende

### Nachweis nach Elektrophorese:

- Zugabe von 5 µl Farbmarker
- je 10 µl auf 2% Hispan - Agarosegel
- Vergleich: 10 µl Marker (λ -DNA;geschnitten mit Hind III)

Die Ergebnisse sind in Abbildung 1 dargestellt. Bei der Elution mit NaOH wurden 90% der DNA wiedergefunden.

In einem weiteren Experiment wurde gezeigt, daß die Abreicherung der DNA sogar in Gegenwart von 1 M Kochsalz in der Proteinlösung erfolgen kann.

Um die Nachweisempfindlichkeit des verwendeten PCR-Protokolls zu belegen, wurde eine Stammlösung λ-DNA (Hind III - Fragmente) in einer Serienverdünnung 1:10 demselben Protokoll unterworfen:

| **Proben - Nr.:** | **1** | **2** | **3** | **4** | **5** | **6** | **7** |
|---|---|---|---|---|---|---|---|
| Mix (µl) | 15 | 15 | 15 | 15 | 15 | 15 | 15 |
| λ Hind III ^{*)} (µl) | 10 | 10 | 10 | 10 | 10 | 10 | 10 |
| DNA - Menge | 100 pg | 10 pg | 1 pg | 100 fg | 10 fg | 1 fg | 100 ag |

| | | | | | | | |
|---|---|---|---|---|---|---|---|
| ^{*}) Ausgangskonzentration 100pg/µl; Reihenverdünnunqen 1 :10; die Verdünnung 7 (100 attogramm) entspricht noch 2 - 3 DNA Molekülen | | | | | | | |

Die Ergebnisse sind in Abbildung 2 dargestellt.

## Patentansprüche

1. Verfahren zur Abtrennung oder Abreicherung von biologischen Kontaminanten aus der Gruppe der Viren, Endotoxine oder Nukleinsäuren aus Lösungen von biologischen Produkten, wobei die aufzureinigende Lösung mit einer Anionenaustauscher-Membran, die erhältlich ist durch Umsetzung eines Polyamides mit einer aminoreaktiven polymerisationsfähigen Verbindung und anschließender Polymerisation mit Monomeren, die kationische Gruppen enthalten, oder in die in polymeranaloger Reaktion kationische Gruppen eingeführt werden können, behandelt wird.

2. Verfahren nach Anspruch 1, wobei Viren die abzutrennende biologische Kontaminante bilden.

3. Verfahren nach Anspruch 1, wobei Nukleinsäuren die abzutrennende biologische Kontaminante bilden.

4. Verfahren nach Anspruch 1, wobei Endotoxine die abzutrennende biologische Kontaminante bilden.

## Claims

1. Process for the removal or reduction in the levels of biological contaminants from the group of viruses, endotoxins or nucleic acids from solutions of biological products, where the solution to be purified is treated with an anion exchanger membrane which is obtainable by reaction of a polyamide with an amino-reactive polymerisation-capable compound followed by polymerisation with monomers containing cationic groups, or into which cationic groups can be introduced in polymer-analogous reaction.

2. Process according to Claim 1, where viruses form the biological contaminant to be removed.

3. Process according to Claim 1, where nucleic acids form the biological contaminant to be removed.

4. Process according to Claim 1, where endotoxins form the biological contaminant to be removed.

## Revendications

1. Procédé pour l'enlèvement ou la réduction dans les niveaux de contaminants biologiques provenant du groupe de virus, d'endotoxines ou d'acides nucléiques de solutions de produits biologiques, dans lequel la solution à purifier est traitée avec une membrane échangeuse d'anions qui peut être obtenue par réaction d'un polyamide avec un composé amino-réactif capable de polymérisation suivie par une polymérisation avec des monomères contenant des groupes cationiques, ou dans laquelle des groupes cationiques peuvent être introduits selon une réaction analogue à un polymère.

2. Procédé selon la revendication 1, dans lequel des virus forment le contaminant biologique à enlever.

3. Procédé selon la revendication 1, dans lequel des acides nucléiques forment le contaminant biologique à enlever.

4. Procédé selon la revendication 1, dans lequel des endotoxines forment le contaminant biologique à enlever.
